# EUROPÄISCHE PATENTSCHRIFT

(11) **EP 2 018 581 B1**
(45) Veröffentlichungstag und Bekanntmachung des Hinweises auf die Patenterteilung: **28.10.2009**
(21) Anmeldenummer: 07725086.8
(22) Anmeldetag: 10.05.2007
(51) Int. Cl.: G01V 8/18, G01P 3/68, G01B 11/24, G01N 25/04

(54) **VORRICHTUNG UND VERFAHREN ZUM ERKENNEN VON OBJEKTEN**
APPARATUS AND METHOD FOR RECOGNIZING OBJECTS
DISPOSITIF ET PROCÉDÉ DE RECONNAISSANCE D'OBJETS

(30) Priorität: 12.05.2006 DE 102006022717
(43) Veröffentlichungstag der Anmeldung: 28.01.2009
(73) Patentinhaber: Büromat It Systeme Zwickau Gmbh, 08060 Zwickau (DE); Petrotest Instruments GmbH & Co. KG, 15827 Dahlewitz (DE)
(72) Erfinder: SCHULZ, Rüdiger, 12249 Berlin (DE); SOMMER, Erik, 15566 Schöneiche (DE); SCHMIDT, Michael, 08459 Neukirchen (DE); WOHLFAHRT, Marko, 09383 Meerane (DE)
(74) Vertreter: Steiniger, Carmen
(86) Internationale Anmeldenummer: PCT/EP2007/004166
(87) Internationale Veröffentlichungsnummer: WO 2007/131708

(56) Entgegenhaltungen:
- DD-A1- 213 061
- JP-A- 61 272 680
- JP-A- 2000 338 262
- US-A- 3 809 891
- "DIN EN 1427:2007: Bitumen und bitumenhaltige Bindemittel - Bestimmung des Erweichungspunktes -Ring- und Kugel-Verfahren" June 2007 (2007-06), DEUTSCHES INSTITUT FÜR NORMUNG EV , BERLIN , XP008082596

## Beschreibung

Die vorliegende Erfindung betrifft eine Vorrichtung und ein Verfahren zum Erkennen von Objekten mit einer Lichtquelle und einem Lichtempfänger.

Eine Vorrichtung dieser Art wird beispielsweise benötigt, um in dem in der europäischen Norm EN 1427 beschriebenen Ring- und Kugel-Verfahren den Erweichungspunkt eines zu testenden Bitumens oder bitumenhaltigen Bindemittels zu bestimmen. Hierbei werden in einem mit einer Flüssigkeit gefüllten lichtdurchlässigen Gefäß auf einer Ringhalterung, auf Bitumenschichtplättchen liegende Stahlkugeln vorgesehen, welche beim kontrollierten Erwärmen der Flüssigkeit im Testverlauf, umhüllt von erweichtem Bitumenmaterial, nach unten fallen und in einem Abstand von 25,0 ± 0,4 mm unterhalb der Ringhalterung detektiert werden. Um die herabfallenden Kugeln an der Messstelle detektieren zu können, werden beispielsweise aus einer LED und einem Lichtempfänger bestehende, am Gefäßrand angeordnete Lichtschranken verwendet. Dieses Prinzip besitzt den Nachteil, dass Störeinflüsse in der Flüssigkeit, wie beispielsweise durch die Erwärmung in dem Gefäß aufsteigende Bläschen oder eine viskositätsabhängige Schichtung in diversen Prüfmedien von der Lichtschranke als Kugel fehlinterpretiert werden können und somit zu unzuverlässigen Ergebnissen führen können.

Die Druckschrift DD 213 061 A beschreibt eine Anordnung, die auf dem Ring- und Kugel-Prinzip basiert. In der Druckschrift ist ausgeführt, dass bei dem bekannten Ring- und Kugel-Verfahren beim Ablauf der Messungen Fehlauslösungen nicht auszuschließen sind, die durch die Ausbildung von Schlieren und Gas- oder Dampfblasen der Temperierbadflüssigkeit oder durch den Einfluss kurzzeitiger Helligkeitsschwankungen der Umgebung infolge Fremdlicht oder der zum Messsystem gehörenden Lichtquelle verursacht werden können. Um eine Messanordnung zu schaffen, mit deren Hilfe ohne zwangsläufige Verwendung eines Rührers eine exakte Erfassung genauer und reproduzierbarer Messwerte für den Erweichungspunkt zweier Proben unter weitestgehender Ausschaltung aller Fehlerquellen und Störeinflüsse möglich ist, schlägt die Druckschrift vor, dass der Lichtschrankeneinheit zur optischen Erfassung der fallenden Kugel ein Schwellenwertindikator mit Filterwirkung, bestehend aus einem elektrischen Tiefpass und einem Trigger, nachgeschaltet wird. Darüber hinaus soll zur Messung, Regelung und Grenzwerterfassung der Temperatur nur ein Temperaturmessfühler verwendet werden, der in der Temperierbadflüssigkeit senkrecht und symmetrisch zu den Proben angeordnet ist und durch seine räumliche Ausbildung bei der Aufheizung entstehende lokale Temperaturinhomogenitäten ausmittelt.

Die Druckschrift JP 2000 338 262 A enthält eine Sensoranordnung zur Detektion der Position bzw. des Durchtrittswinkels eines Objektes. Hierfür wird eine Reflexionsplatte, welche reflektierende und nicht reflektierende Oberflächenbereiche aufweist, mittels Laserlicht abgespannt, das durch eine Linsen-Spiegel-Anordnung auf die Reflexionsplatte gerichtet wird. Die reflektierenden und nicht reflektierenden Oberflächenbereiche sind in regelmäßigen Abständen auf der Reflexionsplatte vorgesehen, sodass das reflektierte Signal eine pulsartige Form aufweist.

Die Druckschrift JP 61 272680 A offenbart ein Detektionsverfahren und eine entsprechende Vorrichtung, mit welchen die Position und die Form eines Körpers bestimmt werden kann. Dabei wird der Körper auf einem Träger befestigt und durch eine Fördervorrichtung transportiert, wobei schwarze und weiße Streifen auf der Oberfläche des Trägers in der Förderrichtung der Fördervorrichtung vorgesehen sind. Ein Laserstrahl wird durch einen Rotationsspiegel derart reflektiert, dass der Träger abgescannt werden kann, wobei das von dem Träger und dem Körper reflektierte Licht durch einen Photodetektor detektiert wird. Das reflektierte Signal ist ein gepulstes Signal, aus welchem die Position und die Länge des Körpers in der Scanrichtung des Laserstrahls und der Bewegungsrichtung des Körpers berechnet werden kann.

Die Druckschrift US 3,809,891 beinhaltet ein Detektionssystem zum Detektieren der Anwesenheit eines Objektes in einem vorgegebenen Bereich. Das Detektionssystem umfasst eine pulsierende Lichtabstrahlvorrichtung bzw. eine lichtemmitierende Diode mit einen nachgeschalteten Pulsgenerator, mit welchen ein Objekt bestrahlt wird, welches sich zwischen der Lichtabstrahlvorrichtung und einem Reflektor bewegt. Das Licht wird durch den Reflektor auf sich selbst reflektiert und über ein Spiegel-Linsen-System zu einer ersten Fotozelle geleitet, wenn nicht der Lichtstrahl durch das Objekt unterbrochen wird. Ferner empfängt eine zweite Fotozelle parallel Lichtimpulse direkt von der Diode der Lichtabstrahlvorrichtung.

Es ist daher die Aufgabe der vorliegenden Erfindung, eine zuverlässige und dennoch leicht anwendbare Vorrichtung und ein entsprechendes Verfahren zum Erkennen von Objekten, wie den oben beschriebenen herabfallenden Kugeln, zur Verfügung zu stellen.

Diese Aufgabe wird durch eine Vorrichtung zum Erkennen von Objekten mit einer Lichtquelle und einem Lichtempfänger gelöst, bei welcher das Objekt in einer in einem lichtdurchlässigen Gefäß vorgesehenen Flüssigkeit nach unten fallbar angeordnet ist, wobei die Lichtquelle eine Laserlichtquelle ist, die Vorrichtung eine wenigstens teilweise reflektierende Wand aufweist und zwischen der Laserlichtquelle und der Wand ein Zwischenraum vorgesehen ist, durch den ein Objekt bewegbar oder in dem ein Objekt platzierbar ist, und im Strahlengang zwischen der Laserlichtquelle und der Wand eine bewegbare Spiegeleinrichtung derart angeordnet ist, dass von der Laserlichtquelle abgestrahltes Licht auf die Wand richtbar ist, wobei die Bewegung der Spiegeleinrichtung so steuerbar ist, dass die Wand wenigstens entlang einer Linie mit dem von der Laserlichtquelle abgestrahlten Licht bestrahlbar ist und von der Wand reflektiertes Licht von dem Lichtempfänger detektierbar ist, und wobei die Wand gewölbt ist, wobei die Wand alternierende helle und dunkle Bereiche, die ein regelmäßiges Streifenmuster ausbilden, aufweist oder das Licht gepulst ist

Mit der erfindungsgemäßen Vorrichtung ist es möglich, eine Änderung der Intensität der von der Wand reflektierten Lichtstrahlung als Indiz zum Erkennen eines zwischen der La-serlichtquelle und der Wand vorhandenen Objektes zu verwenden. Da der von der Laserlichtquelle ausgehende Lichtstrahl eine ganze Linie auf der Wand abtasten kann, können bei entsprechender Abtastfrequenz und Auflösung der vom Lichtempfänger detektierten, reflektierten Lichtstrahlung nicht nur das Objekt selbst sondern auch dessen bei der Messung ermittelte Position auf der Projektionslinie der Laserlichtquelle sowie die Größe des Objektes erfasst werden. Somit kann eine sichere Erkennung bewegter als auch ruhender Objekte mit einer relativ einfachen Anordnung gewährleistet werden.

Es ist ganz besonders von Vorteil, wenn die Wand alternierende helle und dunkle Bereiche aufweist. Mit Hilfe der hellen und dunklen Bereiche auf der Wand können Markierungen gesetzt werden, anhand welcher eine genaue Ermittlung der Größe und der Position des Objektes möglich ist. Bewegt sich beispielsweise ein lichtundurchlässiges Objekt an den hellen und dunklen Bereichen der Wand vorbei oder wird es vor diesem Bereich platziert, werden diese zumindest kurzzeitig abgedeckt, so dass der Lichtempfänger zu diesem Zeitpunkt insbesondere von den hellen Bereichen keine oder eine durch den Störeinfluss verringerte reflektierte Lichtstrahlung aufnehmen kann. Wird beispielsweise von dem Lichtempfänger ein heller Bereich als High-Pegel und ein dunkler Bereich als Low-Pegel erfasst, tritt beim Vorbeibewegen oder Platzieren eines Objektes an einem hellen Bereich beim Lichtempfänger eine Signaländerung auf, bei welcher anstatt eines High-Pegels für die entsprechenden hellen Bereiche der Wand ein Low-Pegel erfasst wird. Auf diese Weise kann die Position des Objektes anhand der Position der durch das Objekt abgedeckten hellen Bereiche festgelegt werden, wobei aus der Anzahl der abgedeckten hellen Bereiche auf die Größe des Objektes geschlossen werden kann.

Es hat sich als besonders günstig erwiesen, wenn die hellen und dunklen Bereiche der Wand ein regelmäßiges Streifenmuster ausbilden. Ein solches Streifen- oder Balkenmuster kann bei dem Lichtempfänger beispielsweise als regelmäßiges 0-1-Bitmuster erfasst werden. Bewegt sich ein Objekt an dem Streifenmuster vorbei oder wird es vor dem Streifenmuster platziert, tritt in dem Bitmuster eine Störung auf, welche sich beispielsweise durch eine Änderung von High-Pegeln in Low-Pegel bemerkbar macht. Dabei kann die Dichte und die Breite der Streifen an die Größe der zu erfassenden Objekte angepasst werden, so dass eine möglichst genaue Identifizierung der jeweiligen Objektgröße möglich ist.

Entsprechend einer weiteren vorteilhaften Variante der Erfindung ist das von der Laserlichtquelle abgestrahlte Licht gepulst. Bei dieser Anordnung können Laserimpulse mit bestimmten Frequenzen verwendet werden, um eine Linie auf der Wand abzurastern, wobei zur Auflösung der zwischen der Laserlichtquelle und der Wand bewegten oder platzierten Objekte die Frequenz der eingesetzten Laserimpulse verwendet werden kann. Somit ist es bei dieser Anordnung für die Objekterkennung nicht unbedingt notwendig, dass die Wand helle und dunkle Bereiche aufweist.

Gemäß einem günstigen Beispiel der Erfindung weist die Spiegeleinrichtung einen elektronisch steuerbaren Schwingspiegel auf. Beispielsweise können rotierende facettierte Spiegel oder auch pendelnde Spiegel eingesetzt werden. Durch die Bewegbarkeit eines solchen Spiegels können punktförmige Laserstrahlen einfach als Linie auf ein Messfeld an der Wand projiziert werden. Vorzugsweise ist während einer Messung die Bewegungsgeschwindigkeit der Spiegeleinrichtung als konstanter Wert eingestellt.

Gemäß einer vorteilhaften Ausführungsform der Erfindung weist der Lichtempfänger einen Lichtsensor auf, der mit einer Auswerteeinheit gekoppelt ist. Die Auswerteeinheit kann beispielsweise von dem Lichtsensor ermittelte Werte in High-Pegel für Hell-Bereiche und Low-Pegel für Dunkel-Bereiche umwandeln, welche Daten mit einem Referenzmuster verglichen werden können, um ein zwischen der Laserlichtquelle und der Wand bewegtes oder platziertes Objekt zu erkennen.

Vorzugsweise weist die Auswerteeinheit eine Kontrastregelungseinheit auf. Hiermit kann eingestellt werden, welche Intensität der von dem Lichtempfänger empfangenen Lichtstrahlung als heller Bereich oder als dunkler Bereich einzuordnen ist. So ist es beispielsweise möglich, dass ein reflektierender Bereich der Wand durch ein davortretendes Objekt lediglich diffus Licht reflektiert, so dass zwar reflektierte Lichtstrahlung aus diesem Bereich von dem Lichtempfänger empfangen wird, aber das empfangene Signal eine geringe Intensität aufweist. In solchen Fällen kann mit Hilfe der Kontrastregelungseinheit der Kontrast so eingestellt werden, dass Signale mit geschwächter Intensität als Low-Pegel eingeordnet werden.

Gemäß einer bevorzugten Ausführungsvariante der vorliegenden Erfindung sind die Laserlichtquelle und der Lichtempfänger in einem Scanmodul mit einem uncodierten High-Low-Ausgangssignal zusammengefasst. Somit können die Laserlichtquelle und der Lichtempfänger praktikabel in einem Gerät untergebracht werden, wodurch die Messvorrichtung für einen Anwender vereinfacht wird. Auch kann auf diese Weise die Ausrichtung der Laserlichtquelle zu dem Lichtempfänger genau und fest aufeinander abgestimmt werden. Das uncodierte Ausgangssignal ermöglicht es zudem auf einfache Weise, Objekte im Erfassungsbereich des Scanmoduls zu detektieren.

Vorteilhafterweise weist die Vorrichtung eine Zeit- und/oder Temperaturerfassungseinheit auf. Somit kann die Zeit und/oder die Temperatur, bei welcher das Objekt von der erfindungsgemäßen Vorrichtung erfasst wurde, ermittelt werden, wobei die Objekterkennung mit der Zeit- und/oder Temperaturerkennung logisch verknüpft werden kann.

In einer speziellen Ausgestaltung der vorliegenden Erfindung ist das bewegte Objekt eine Kugel, welche in einer in einem lichtdurchlässigen Gefäß vorgesehenen Flüssigkeit nach unten fallbar angeordnet ist, wobei um das Gefäß die Laserlichtquelle, der Lichtempfänger, die Spiegeleinrichtung und die Wand angeordnet sind. Eine solche Anordnung kann verwendet werden, um anhand des Zeitpunktes, an dem die Kugel erfasst wird, Rückschlüsse darauf zu ziehen, zu welchem Zeitpunkt der Fall der Kugel eingesetzt hat. Ebenso kann diese Vorrichtung verwendet werden, um eine Bewegungsgeschwindigkeit einer Kugel in einer Flüssigkeit zu ermitteln.

Gemäß einer besonders bevorzugten Ausführungsform der Erfindung wird die Kugel auf einer erweichbaren Schicht in einer Ringhalterung gehalten, unter welcher im Abstand von 25,0 ± 0,4 mm ein Messbereich zum Erfassen der Kugel vorgesehen ist. Diese Anordnung eignet sich insbesondere für die Verwendung der Vorrichtung in dem so genannten Ring- und Kugel-Verfahren, in welchem der Erweichungspunkt von Bitumen- oder bitumenhaltigem Bindemittelschichten bestimmt wird. So könnte beispielsweise eine Anordnung verwendet werden, welche in der europäischen Norm EN 1427:1999 beschrieben ist.

Die Aufgabe der Erfindung wird ferner durch ein Verfahren zum Erkennen bewegter Objekte mit einer Lichtquelle und einem Lichtempfänger gelöst, bei welchem ein in einer in einem lichtdurchlässigen Gefäß vorgesehenen Flüssigkeit herabfallendes Objekt erfasst wird, wobei die Lichtquelle eine Laserlichtquelle ist, von welcher Licht auf eine bewegbare Spiegeleinrichtung gerichtet wird, wobei die Spiegeleinrichtung das Licht auf eine gewölbte, zumindest teilweise reflektierende Wand richtet und die Spiegeleinrichtung dabei so gesteuert wird, dass die Wand wenigstens entlang einer Linie durch das Licht bestrahlt wird, und wobei der Lichtempfänger das von der Wand reflektierte Licht detektiert und dabei ein sich zwischen der Laserlichtquelle und der Wand bewegendes oder platziertes Objekt durch eine Änderung des detektierten, reflektierten Lichts erfasst, und wobei ein auf der Wand vorgesehenes, regelmäßiges Streifenmuster von dem Lichtempfänger mit einer Auswerteeinheit als kontinuierliches, uncodiertes 0-1-Bitmuster erfasst wird oder das Licht gepulst auf die Spiegeleinrichtung gestrahlt wird.

Mit dem erfindungsgemäßen Verfahren können Objekte zwischen der Laserlichtquelle und der Wand entlang der gesamten von dem Laserlicht erfassten Linie erkannt werden, wobei die Lichtquelle und der Lichtempfänger auf der gleichen Seite der Vorrichtung, gegenüber der Wand, angeordnet werden können, was es möglich macht, die Lichtquelle und den Lichtempfänger in einem Scanmodul zu integrieren. Mit Hilfe dieses Verfahrens können somit Objekte auf einfache Weise sicher erfasst werden.

Gemäß einer bevorzugten Ausführungsform der Erfindung ist der Lichtempfänger mit einer Auswerteeinheit gekoppelt, welche in einem Ausgangssignal für helle Bereiche der Wand einen High-Pegel und für dunkle Bereiche der Wand einen Low-Pegel ausgibt Somit können helle und dunkle Bereich der Wand genutzt werden, um anhand von Intensitätsänderungen des von dem Lichtempfänger empfangenen, reflektierten Laserlichts an der Wand vorbei bewegte oder dort platzierte Objekte zu erkennen.

Hierbei ist es besonders günstig, wenn ein auf der Wand vorgesehenes, regelmäßiges Streifenmuster von dem Lichtempfänger mit der Auswerteeinheit als kontinuierliches, uncodiertes 0-1-Bitmuster erfasst wird. Dabei bildet das uncodierte 0-1-Bitmuster ohne ein sich zwischen der Lichtquelle und der Wand befindliches Störobjekt ein geeignetes Referenzmuster aus, das es ermöglicht, bei dessen Störung durch ein Objekt nicht nur das Objekt selbst sondern auch dessen Größe zu erfassen.

Es ist besonders von Vorteil, wenn das Ausgangssignal durch das Objekt so gestört wird, dass in dem Bereich, in dem sich das Objekt bewegt oder platziert ist, anstelle von High-Pegeln Low-Pegel erfasst werden. Hierdurch ist auf einfache, aber eindeutige Weise eine genaue Aussage dahingehend möglich, ob sich ein Objekt zwischen der Lichtquelle und der Wand befindet oder nicht.

Gemäß einer weiteren, ebenfalls sehr vorteilhaften Ausführungsform der Erfindung wird das Licht von der Laserlichtquelle gepulst auf die Spiegeleinrichtung gestrahlt. Damit kann das Laserlicht von der Spiegeleinrichtung gepulst auf die Wand projiziert werden und das von der Wand reflektierte Licht in entsprechenden Impulsen von dem Lichtempfänger erfasst werden, wobei anhand der verwendeten Frequenz genau ermittelt werden kann, zu welchem Zeitpunkt und an welcher Stelle das Objekt zwischen der Laserlichtquelle und der Wand detektiert wurde.

In einem speziellen Beispiel der Erfindung wird eine in einer Flüssigkeit in einem lichtdurchlässigen Gefäß herabfallende Kugel erfasst. Mit diesem Verfahren kann anhand des Erfassungszeitpunktes der Kugel auf die Zeit des Beginns ihres Falls in dem Gefäß geschlossen werden. Es ist auch möglich, mit diesem Verfahren die Bewegungsgeschwindigkeit der Kugel in der Flüssigkeit zu ermitteln.

Gemäß einem besonders bevorzugten Beispiel der Erfindung ist die Kugel eine Stahlkugel, die vor dem Herabfallen auf einer Schicht in einer Ringanordnung aufliegt, wobei die Schicht bei einer bestimmten Temperatur erweicht wird und die vom Schichtmaterial umhüllte Kugel nach unten fällt, wobei die Kugel nach einer bestimmten Fallstrecke erfasst wird. Dieses Verfahren eignet sich besonders gut bei der Verwendung in dem so genannten Ring- und Kugel-Verfahren zur Bestimmung des Erweichungspunktes von Bitumen oder bitumenhaltigen Bindemitteln, welches beispielsweise in der europäischen Norm EN 1427:1999 beschrieben ist, auf welche hier Bezug genommen wird.

Eigenschaften, die Funktionsweise und Vorteile der vorliegenden Erfindung werden im folgenden anhand der Figuren der Zeichnung beschrieben, in welchen
- Figur 1: schematisch das Funktionsprinzip einer ersten Ausführungsform der vorliegenden Erfindung zeigt;
- Figur 2: schematisch das Funktionsprinzip der Vorrichtung von Figur 1 anhand einer Ring-Kugel-Messanordnung in der Draufsicht zeigt;
- Figur 3: schematisch die Anordnung aus Figur 2 in der Seitenansicht zeigt;
- Figur 4: schematisch ein Streifenmuster ohne Störobjekt zeigt;
- Figur 5: ein Bitmuster ohne Störobjekt entsprechend dem Streifenmuster aus Figur 4 zeigt;
- Figur 6: schematisch ein Streifenmuster mit Störobjekt zeigt;
- Figur 7: ein Bitmuster mit Störobjekt entsprechend dem Streifenmuster aus Figur 6 zeigt;
- Figur 8: ein Beispiel für ein Signal eines Scanmoduls zeigt, mit welchem der zeitliche Verlauf des Scanvorgangs bestimmt wird;
- Figur 9: ein Beispiel für eine Ausgangssignal mit High- und Low-Pegeln eines Scanmoduls zeigt;
- Figur 10: ein Blockdiagramm zeigt, das die I²C-Handler-Funktion darstellt;
- Figur 11: die für eine Initialisierung des Scanmoduls erforderlichen Schritte anhand eines Blockdiagramms zeigt;
- Figur 12: die zum Scannen einer Zeile erforderlichen Schritte anhand eines Blockdiagramms zeigt;
- Figur 13: die Schritte eines INT-Interrupts beim Erkennen eines nächsten Balkens in einem Blockdiagramm zeigt;
- Figur 14: in einem Blockdiagramm die Schritte eines Timer "0"-Überlaufinterrupts zeigt; und
- Figur 15: schematisch das Funktionsprinzip einer weiteren Ausführungsform der vorliegenden Erfindung zeigt

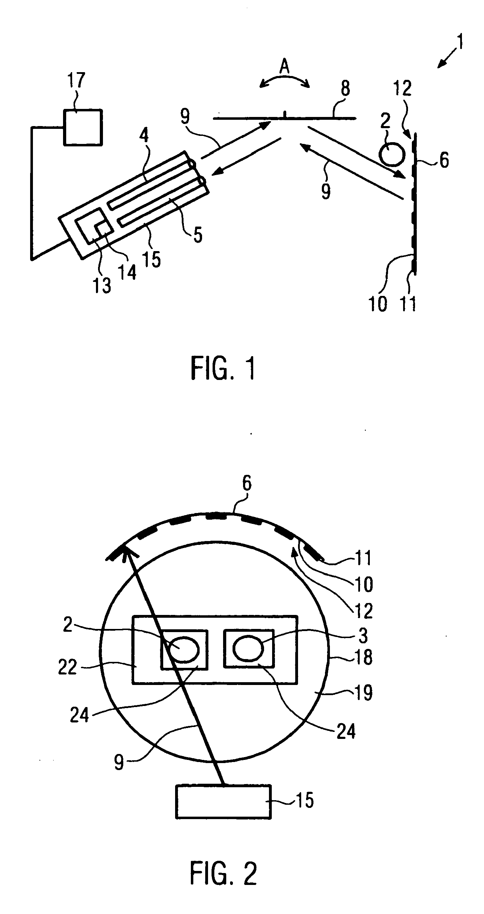

Figur 1 zeigt schematisch das Funktionsprinzip einer erfindungsgemäßen Vorrichtung 1 zum Erkennen eines zwischen einer Laserlichtquelle 4 und einer Wand 6 bewegten Objektes 2.

In der Vorrichtung 1 ist die Laserlichtquelle 4 und ein Lichtempfänger 5 gegenüber der Wand 6 angeordnet, wobei Licht 9, das von der Laserlichtquelle 4 angestrahlt wird, zunächst auf eine Spiegeleinrichtung 8 gerichtet wird und von der Spiegeleinrichtung 8 auf die Wand 6 projiziert wird. Hierfür ist die Laserlichtquelle 4 im Winkel zu der Spiegelfläche der Spiegeleinrichtung 8 angeordnet.

Die Spiegeleinrichtung 8 umfasst einen bewegbaren, elektronisch steuerbaren Schwingspiegel, welcher entsprechend der durch den Pfeil A angedeuteten Bewegungsrichtungen schwenkbar ist. Die Spiegeleinrichtung 8 ist mit einem elektronischen Steuermodul gekoppelt, welches in Figur 1 nicht dargestellt ist. Die Spiegeleinrichtung 8 kann beispielsweise ein rotierender facettierter Spiegel oder auch ein pendelnder Spiegel sein.

Die Spiegeleinrichtung 8 projiziert einen von der Laserlichtquelle 4 abgestrahlten punktförmigen Laserstrahl 9 durch ihre Bewegung als Linie auf ein Messfeld auf der Wand 6.

Die Wand 6 ist in Figur 1 als ebene Fläche dargestellt, ist jedoch gemäß der Erfindung, wie in Figur 2 gezeigt, als gekrümmte Fläche ausgebildet. Die der Laserlichtquelle 4 zugewandte Oberfläche der Wand 6 ist zumindest teilweise reflektierend. In dem in Figur 1 gezeigten Beispiel weist die Wand 6 auf der der Laserlichtquelle zugewandten Seite helle Bereiche 10 und dunkle Bereiche 11 auf, weiche in dem gezeigten Beispiel ein regelmäßiges Streifenmuster 12 ausbilden

Gegenüber der Wand 6 ist ein Lichtempfänger 5 für das von der Wand 6 reflektierte Licht 9 angeordnet. Der Lichtempfänger 5 weist die gleiche Ausrichtung wie die Laserlichtquelle 4 auf, so dass er von der Wand 6 reflektiertes Licht 9, das auf die Spiegeleinrichtung 8 fällt, über die Spiegeleinrichtung 8 empfangen kann. Der Lichtempfänger 5 ist mit der Laserlichtquelle 4 in einem Scanmodul 15 zusammengefasst.

Der Lichtempfänger 5 weist einen Lichtsensor auf, der mit einer Auswerteeinheit 13 für die empfangenen Lichtsignale gekoppelt ist. In der Auswerteeinheit 13 ist eine Kontrastregelungseinheit 14 mit einem Schwellwertschalter zum Einstellen des Kontrastes des empfangenen Lichtes 9 vorgesehen.

Die Vorrichtung 1 weist zudem eine Zeit- und Temperaturerfassungseinheit 17 auf, welche mit der Auswerteeinheit 13 gekoppelt ist. In andren, nicht gezeigten Ausführungsformen der Erfindung können Zeit und Temperatur auch mit voneinander unabhängigen Messeinrichtungen zum Erfassen der Zeit oder der Temperatur erfasst werden.

Wird von der Laserlichtquelle 4 Laserlicht 9 auf die Spiegeleinrichtung 8 gestrahlt, projiziert die Spiegeleinrichtung 8 den Laserstrahl 9 durch ihre Bewegung in einer Linie auf die Wand 6, wobei die dabei erzeugte Lichtlinie die auf der Wand 6 vorgesehenen Streifen 12 etwa im rechten Winkel schneidet. Durch das regelmäßige Streifenmuster 12 werden durch die Wand 6 in regelmäßigen Abständen Lichtstrahlen 9 reflektiert bzw. nicht oder diffus reflektiert. Auf diese Weise empfängt der Lichtsensor des Lichtempfängers 5 Lichtsignale mit höherer und Lichtsignale mit geringerer Intensität oder für bestimmte Bereich gar kein Lichtsignal.

Durch die Auswerteeinheit 13 werden die Lichtsignale höherer Intensität als High-Pegel und Lichtsignale mit geringerer Intensität als Low-Signale aufgenommen und in einem High-Low-Ausgangssignal 16 zusammengefasst. Das High-Low-Ausgangssignal 16 kann als ein 0-1-Bitmuster 23 erfasst werden. Ein ungestörtes 0-1-Bitmuster 23 kann als Referenzmuster für einen Vergleich mit einem Bitmuster mit einem Störobjekt 2 verwendet werden.

Die in der Auswerteeinheit 13 vorgesehene Kontrastregelungseinheit 14 kann den Kontrast des von dem Lichtsensor erfassten Lichtes so einstellen, dass diejenigen Signale, die über einem bestimmten Schwellwert liegen, als High-Signale eingeordnet werden, und die Signale, die unter diesem Schwellwert liegen, als Low-Signale eingeordnet werden. Hierfür weist die Kontrastregelungseinheit 14 einen Schwellwertschalter auf, mit welchem eine Anpassung an die Kontrastverhältnisse zwischen dem Referenzhintergrund auf der Wand 6 und einem davor bewegten oder platzierten Objekt 2 vorgenommen werden kann.

Bewegt sich ein Objekt 2 in einem Zwischenraum zwischen der Laserlichtquelle 4 und der Wand 6 an der durch die Spiegeleinrichtung 8 auf die Wand 6 projizierten Linie vorbei oder wird es dort platziert, werden zumindest kurzzeitig die von dem Lichtempfänger 5 erfassten Lichtsignale verändert. Durch das Objekt 2 werden Unregelmäßigkeiten in dem von der Auswerteeinheit 13 erzeugten Bitmuster 23 erzeugt, da das Objekt 2, zumindest wenn es lichtundurchlässige Bereiche besitzt, eine Reflexion der hellen Bereiche 10 der Wand 6 verhindert oder verringert, so dass die Intensität der von den hellen Bereichen 10 der Wand 6 reflektierten Lichtstrahlen durch das Objekt 2 verringert wird. Entsprechend werden an der Stelle, an welcher sich das Objekt 2 an der Wand 6 vorbeibewegt hat bzw. an welcher es sich befindet, anstelle von High-Pegeln, welche den hellen Bereichen 10 der Wand 6 entsprechen, zumindest kurzzeitig Low-Pegel von dem Lichtempfänger 5 erfasst werden.

Ist das Streifenmuster 12 auf der Wand 6 ausreichend dicht und sind die Streifen und die dazwischen befindlichen Abstände entsprechend schmal, können durch das Objekt 2 mehrere helle Bereiche 10 abgedeckt werden, so dass an der Stelle, an der das Objekt 2 sich an der Wand 6 vorbeibewegt hat oder an der es platziert ist, eine Reihe von Low-Pegeln bzw. 0-Werten in dem 0-1-Bitmuster 23 ermittelt wird. Aus der Anzahl der Low-Pegel bzw. der 0-Werte ist dann ableitbar, wie groß das Objekt 2 ist. Zudem kann von der Position der ermittelten Bitmusterstörung auf die Position des Objektes 2 geschlossen werden.

Die oben beschriebene Vorrichtung und das Verfahren können zum Erfassen bewegter und ruhender Objekte zwischen der Laserlichtquelle 4 und der Wand 6 verwendet werden.

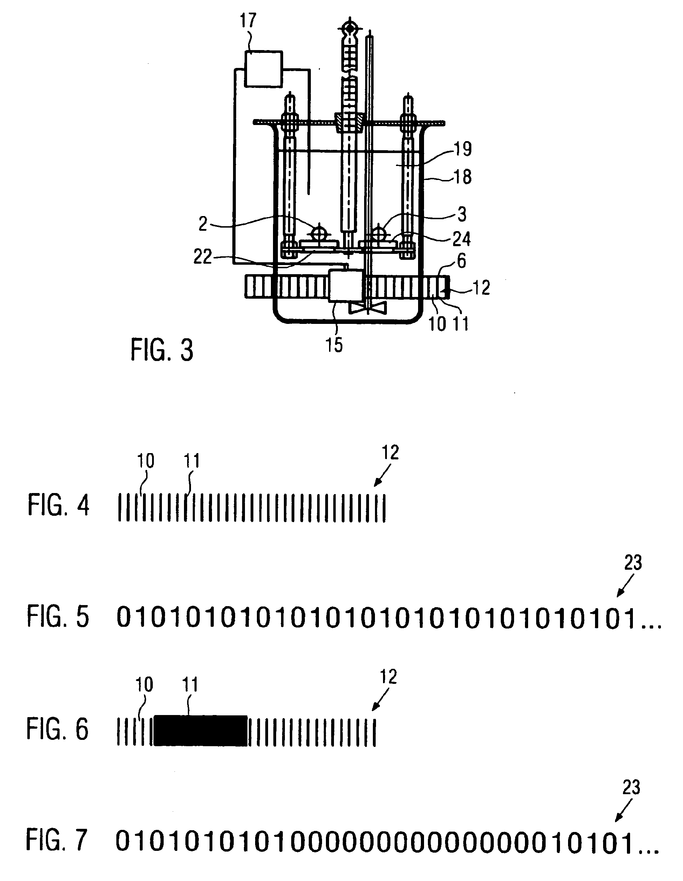

Figur 2 zeigt schematisch das anhand von Figur 1 beschriebene Funktionsprinzip der erfindungsgemäßen Vorrichtung 1 anhand einer speziellen Ausführungsvariante der Erfindung in der Draufsicht und Figur 3 zeigt diese Ausführungsform in der Seitenansicht.

In der in Figur 2 und 3 gezeigten Ausführungsform der Erfindung befinden sich zwei zu messende Objekte 2, 3 in einem lichtdurchlässigen Becherglas 18, das mit einer lichtdurchlässigen Flüssigkeit 19 gefüllt ist. Die zu messenden Objekte 2, 3 sind hier zwei Stahlkugeln, welche jeweils auf aus Bitumenplättchen 24 bestehenden Schichten aufliegen, die in einer Ringhalterung 22 gehalten werden.

Um das Becherglas 18 ist auf einer Seite des Gefäßes 18 unterhalb der Ringhalterung eine gewölbte Wand 6 vorgesehen, auf welcher ein senkrechtes Streifenmuster 12 abgebildet ist.

Der Wand 6 gegenüber befindet sich auf der anderen Seite des Becherglases 18 ein Scanmodul 15 mit einer Laserlichtquelle 4, einer Spiegeleinrichtung 8 und einem Lichtempfänger 5. Durch die in dem Scanmodul 15 vorgesehene Spiegeleinrichtung 8 wird ein von der Laserlichtquelle 4 ausgehender Laserstrahl 9 so auf die Wand 6 projiziert, dass sich auf der Wand 6 eine das Streifenmuster 12 schneidende Laserlichtlinie ergibt.

Das von den hellen Bereichen 10 der Wand reflektierte Laserlicht wird durch den Lichtempfänger 5 mittels der Spiegeleinrichtung 8 in dem Scanmodul 15 empfangen. Dabei bildet sich in der Auswerteeinheit 13 des Scanmoduls 15 zunächst, das heißt ohne ein zwischen dem Scanmodul 15 und der Wand 6 befindliches Objekt, ein regelmäßiges 0-1-Bitmuster 23 ab, welches den regelmäßigen Streifen des Streifenmusters 12 auf der Wand 6 entspricht.

Zu Beginn der Messung wird mit einer Zeit- und/oder Temperaturerfassungseinheit 17 der Beginn der Messung mit t = 0 festgelegt. Daraufhin wird die Flüssigkeit 19 kontinuierlich unter ständigem Rühren der Flüssigkeit 19 erwärmt und die Temperatur gemessen, bis die Bitumenplättchen 24, auf welchen die Kugeln 2, 3 aufliegen, erweichen, woraufhin die Kugeln 2, 3 umhüllt vom Bitumenmaterial 24 durch die Ringhalterung 22 hindurch fallen.

Erreichen die Kugeln 2, 3 den Messbereich, der sich 25 ± 0,4 mm unterhalb der Ringhalterung 22 befindet, werden die Kugeln 2, 3 durch das Scanmodul 15 erfasst, wobei die Zeit- und Temperaturerfassungseinheit 17 den zugehörigen Zeitpunkt t₂ bzw. t₃ sowie die entsprechenden Temperaturen T₂ bzw. T₃ erfasst.

Zu den Zeitpunkten, an welchen die Kugeln 2, 3 vor dem Streifenmuster 12 der Wand 6 herabfallen, werden jeweils helle Bereiche 10 des Streifenmusters 12 abgedeckt, so dass der Lichtempfänger 5 in dem Scanmodul 15 von diesen Bereichen ein Low-Signal erhält. Aus der Anzahl der Low-Signale ermittelt die Auswerteeinheit 13 in dem Scanmodul 15 die Größe des herabgefallenen Objektes und kann daraufhin feststellen, ob es sich tatsächlich um eine der Kugeln 2, 3 handelt oder ob beispielsweise die Größe des ermittelten Objektes eher darauf hinweist, dass das erkannte Objekt eine in dem Gefäß 18 aufsteigende Blase ist.

Die Auswerteeinheit 13 weist eine Software auf, welche in Abhängigkeit von der verwendeten Frequenz der Laserlichtquelle 4, dem Typ der Laserlichtquelle 4, dem Abstand des Scanmoduls 15 und der Wand 6 von dem Gefäß 18, der Zusammensetzung der Flüssigkeit 19, dem Brechindex der Wand des Gefäßes 18 und/oder weiterer konstruktiver oder versuchsspezifischer Merkmale die erfassten Daten verarbeitet.

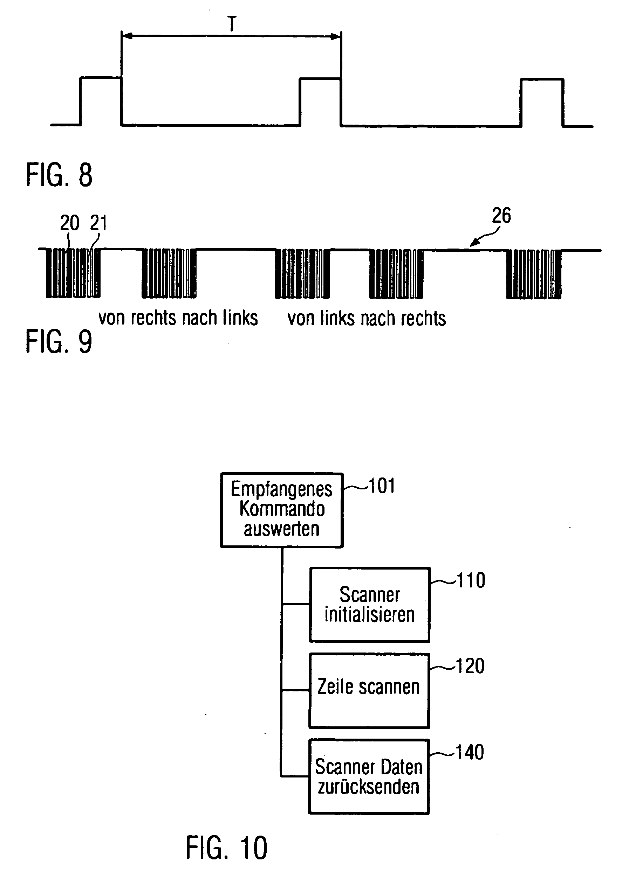

Das in der Vorrichtung von Figur 2 und 3 verwendete Scanmodul 15 ist ein Non-Decoded-Scanengine. Das heißt, das Scanmodul 15 liefert lediglich ein digitalisiertes Signal seiner Abtastlogik, setzt das eingelesene Muster jedoch nicht in einen bestimmen Barcodesatz um. Die Auswerteeinheit 13 des Scanmoduls 15 nutzt zwei digitale Signale, über welche der zeitliche Verlauf eines Scanzyklus sowie das eingelesene Muster bestimmt werden können, wobei Beispiele solcher Signale in den Figuren 8 und 9 dargestellt sind. Wichtig bei der in Figur 2 und 3 gezeigten Ausführungsform ist, dass dem Scanmodul 15 als Hintergrund auf der Wand 6 ein Muster mit scharfen Hell-Dunkel-Übergängen, ähnlich dem eines Barcodes, vorliegt, so dass die Funktionalität des Scanmoduls 15 gewährleistet ist.

Figur 4 zeigt schematisch ein Beispiel für das in Figur 3 verwendete Streifenmuster 12 ohne Störobjekt 2, 3. Das Streifenmuster 12 weist alternierende helle und dunkle Bereiche 10, 11 auf, die im regelmäßigen Abstand zueinander angeordnet sind.

Figur 5 zeigt ein Bitmuster 23 für das Streifenmuster 12 aus Figur 4 ohne Störobjekt 2, 3. Entsprechend dem regelmäßigen Streifenmuster 12 ergibt sich ein regelmäßiges 0-1-Bitmuster 23 ohne Störungen, das als Referenzmuster eingesetzt werden kann.

Figur 6 zeigt ein Beispiel für ein Streifenmuster 12 mit einem Störobjekt 2. An der Stelle, an welcher sich das Objekt 2 an dem Streifenmuster vorbeibewegt hat oder an welcher es platziert wurde, sind keine hellen Bereiche 10 zu erkennen.

Figur 7 zeigt ein Bitmuster 23, welches dem Streifenmuster 12 mit Störobjekt 2 aus Figur 6 entspricht. An der Stelle, an welcher eine Störung durch das Objekt 2 erfolgte, weist das Bitmuster 23 eine Reihe von 0-Pegeln auf.

Figur 8 zeigt ein Beispiel eines Signals einer Auswerteeinheit 13, mit welcher der zeitliche Verlauf des Scanvorgangs bestimmt werden kann. Das in Figur 8 gezeigte Signal liefert dabei bei Beginn des Scanzyklus eine High-Low-Flanke, wobei in dem hier dargestellten Beispiel ein Messzyklus eine Dauer T von etwa 33 Millisekunden bis etwa 39 Millisekunden aufweist. Ein Scanzyklus besteht dabei aus einem Laserlauf von rechts nach links und wieder zurück.

Figur 9 zeigt ein Beispiel für ein Ausgangssignal 16 einer Auswerteeinheit 13, wobei das dargestellte Signal ein eingelesenes Muster ausgibt. Ein Low-Signal signalisiert eine dunkle, weniger reflektierende Tastregion und ein High-Signal ist einem hellen, stark reflektierenden Bereich zuzuordnen.

Vorzugsweise besteht das auf der Wand 6 abgebildete Hintergrundmuster aus schwarzen Balken einer festen Breite, welche einen konstanten Abstand zueinander besitzen. Befindet sich kein Objekt 2, 3 zwischen dem Scanmodul 15 und dem Balkenmuster 12, ist auf einer Dataout-Leitung des Scanmoduls 15 ein regelmäßiges High-Low-Ausgangssignal 16 zu verzeichnen. Wird ein Gegenstand bzw. ein Objekt 2, 3 in das Laserfeld des Scanmoduls 15 eingeführt, wird das Balkenmuster 12 unterbrochen, was zu einer Änderung des Dataout-Signals führt. Je nach Reflexionseigenschaften des Objektes 2, 3 weist das Signal an dieser Stelle entweder ein verlängertes Low- oder High-Signal auf. Diese Musterunterbrechung wird nun mit Hilfe der ein der Auswerteeinheit 13 vorgesehenen Auswerteelektronik detektiert und ausgewertet.

Figur 10 zeigt schematisch in einem Blockdiagramm die von einem I²C-Handler für das Scanmodul 15 zu koordinierende Schritte. Entsprechend Schritt 101 ist zunächst ein empfangenes Kommando auszuwerten. Im Schritt 110 ist das Scanmodul 15 zu initialisieren. Im Schritt 120 wird von dem Scanmodul 15 eine Zeile auf der Wand 6 gescannt. Im Schritt 140 werden die Scannerdaten an die Auswerteeinheit 13 zurückgegeben. Die gescannten Signale werden von einem PIC-Controller eingelesen, das eingelesene Muster in geeigneter Form abgespeichert und dieses einem Hostcontroller zur weiteren Auswertung über ein I²C-Businterface angeboten.

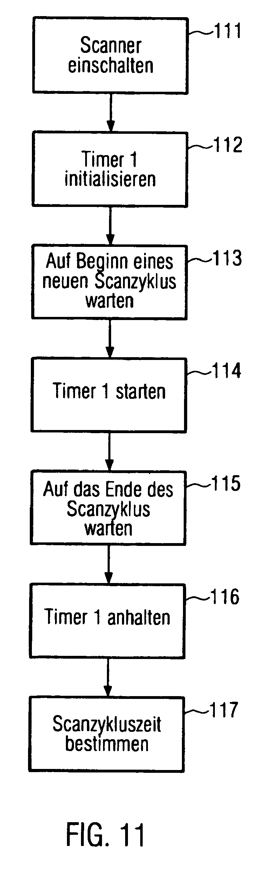

Figur 11 zeigt schematisch anhand eines Blockdiagramms, wie die Scannerlogik des Scanmoduls 15 mit Hilfe eines speziellen I²C-Befehls initialisiert wird. Hierbei wird im Schritt 111 das Scanmodul 15 zunächst eingeschalten und eine bestimmte Einschwingzeit des Lasermechanismus abgewartet. Im Schritt 112 wird ein Timer "1" initialisiert. Im Schritt 113 wird auf den Beginn eines neuen Scanzyklus gewartet. Im Schritt 114 wird der Timer "1" gestartet.

Entsprechend Schritt 115 wird auf das Ende des Scanzyklus gewartet. Im Schritt 116 wird der Timer "1" angehalten und im Schritt 117 wird die Scanzykluszeit bestimmt und im PIC-Controller gespeichert. Das Scanmodul 15 bleibt danach eingeschaltet.

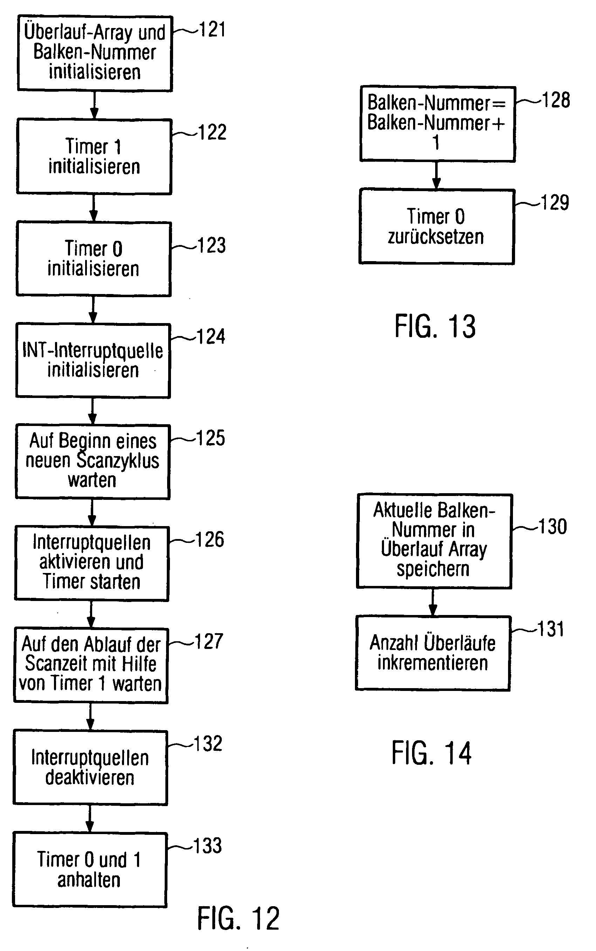

Figur 12 zeigt schematisch anhand eines Blockdiagramms die im Schritt 120 für das Scannen einer Zeile ausgeführten Teilschritte. Sendet der Hostcontroller ein Kommando zum Einlesen einer Scanzeile an den PIC-Controller, so beginnt dieser mit der Mustererkennungsroutine. Im Schritt 121 werden ein Überlauf-Array und eine Balkennummer initialisiert. Entsprechend Schritt 122 wird der Timer "1" initialisiert. Im Schritt 123 wird ein interner Timer, der Timer "0", initialisiert. Des Weiteren wird in der Mustererkennungsroutine das Dataout-Signal des Scanmoduls 15 als Interruptquelle (INT) zu Hilfe genommen, welche im Schritt 124 initialisiert wird. Im Schritt 125 wird zunächst gewartet, bis ein neuer Scanzyklus beginnt. Daraufhin werden im Schritt 126 der Timer "0" und die Interruptquelle aktiviert. Zusätzlich wird im Schritt 127 der Timer "1", gestartet, welcher nach der Hälfte der Scanzykluszeit einen Interrupt liefert. Tritt dieser Interrupt auf, so wird das Einlesen beendet, so dass die eingelesenen Daten nur das Muster für eine Richtung des Lasers, beispielsweise von rechts nach links, wiedergeben.

Der Timer "0" wird so eingestellt, dass er nach einer bestimmten Zeit, welche viel kleiner als die Gesamtzykluszeit ist, einen Überlaufinterrupt auslöst. Vor dem Start der Mustererkennungsroutine wird nun ein Zähler, welcher eine aktuelle Balkennummer enthält, zurückgesetzt. Tritt ein INT-Interrupt, das heißt eine High-Low-Flanke des Dataout-Signals, auf, so befindet sich der Laser des Scanmoduls 15 bei einem neuen Balken des Streifenmusters 12. Wie in Figur 13 gezeigt, wird im Schritt 128 die aktuelle Balkennummer in der zugehörigen Interruptroutine inkrementiert und im Schritt 129 der Timer "0" zurückgesetzt. Damit kann gewährleistet werden, dass bei einem regelmäßigen Balkenmuster 12 der Überlaufinterrupt des Timers "0" nicht auftritt. Ist das Streifenmuster 12 jedoch unterbrochen, so tritt der Überlauf auf und es wird eine entsprechende Interruptroutine, wie in Figur 14 gezeigt, ausgeführt. Dabei wird im Schritt 130 die aktuelle Balkennummer in einem Überlaufarray gespeichert und im Schritt 131 die Anzahl der Überläufe inkrementiert. Tritt der Überlauf mehrfach bei einer Balkennummer auf, so wird die Balkennummer auch mehrfach im Array gespeichert.

Ist das Einlesen beendet, so kann der Hostcontroller die eingelesenen Daten vom PIC-Controller abfragen und für eine weitere Auswertung heranziehen. Er erhält dabei den Inhalt des Überlaufarrays, dessen Größe sowie die Nummer des letzten Balkens.

Die gespeicherten Balkennummern im Überlaufarray im Zusammenhang mit der Anzahl der Gesamtbalken, das heißt der Nummer des letzten Balkens, sind nun ein Indikator für die Länge und Position einer Unterbrechung innerhalb des Balkenmusters 12. Es ist dabei unerheblich, ob das Objekt 2, 3, welches die Unterbrechung auslöste, zu einem verlängerten Low- oder High-Signal 16 der Dataout-Leitung führte. Im Schritt 132 von Figur 12 werden die Interruptquellen deaktiviert und im Schritt 133 die Timer "0" und "1" angehalten.

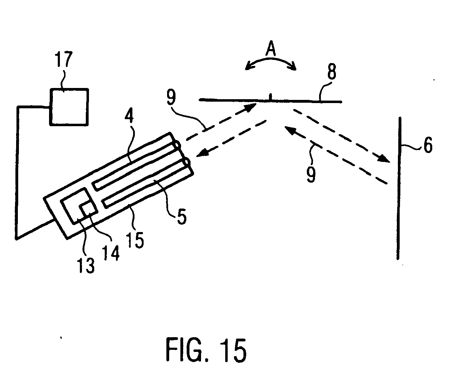

Figur 15 zeigt schematisch eine weitere mögliche Ausführungsvariante der erfindungsgemäßen Vorrichtung 1'.

Da die Vorrichtung 1' gleiche bzw. ähnliche Elemente wie die Vorrichtung 1 beinhaltet, werden im folgenden gleiche Bezugszeichen für gleiche Elemente wie in Vorrichtung 1 verwendet, wobei auf die oben erfolgte Beschreibung dieser Elemente verwiesen wird.

Wie die Vorrichtung 1 weist die Vorrichtung 1' ein gegenüber einer zumindest teilweise reflektierenden Wand 6 gegenüber angeordnetes Scanmodul 15 mit einer Laserlichtquelle 4 und einem Lichtempfänger 5 sowie einer Auswerteeinheit 13 auf. Zwischen dem Scanmodul 15 und der Wand 6 ist eine drehbare Spiegeleinrichtung 8 vorgesehen, mit welcher Laserlicht 9 auf die Wand 6 projiziert werden kann, wobei von der Wand 6 reflektiertes Laserlicht von dem Lichtempfänger 5 empfangen wird.

Im Unterschied zu der Vorrichtung 1 weist die Wand 6 der Vorrichtung 1' kein Streifenmuster 12 auf. Die von der Laserlichtquelle 4 abgestrahlten Laserstrahlen 9 sind jedoch gepulst. Wird ein Objekt 2, 3 zwischen der Laserlichtquelle 4 und der Wand 6 bewegt oder in dem Zwischenraum zwischen der Laserlichtquelle 4 und der Wand platziert, ändert sich die Intensität des von dem Lichtempfänger 5 empfangenen Lichtes. Die Stelle, an der das Objekt 2, 3 detektiert wurde, kann in der Vorrichtung 1' unter Verwendung der eingesetzten Frequenz der Laserimpulse ermittelt werden. Beispielsweise kann hierfür eine Frequenz von 10 kHz eingesetzt werden.

Auch mit der Vorrichtung 1' ist es möglich, nicht nur das Objekt 2, 3 selbst sondern auch unter Verwendung einer Zeit- und Temperaturerkennungseinheit 17 die Zeit und die Temperatur zu erfassen, bei welcher das Objekt erkannt wurde. Auf diese Weise kann ein kompletter Bewegungsablauf eines Objektes nachvollzogen werden. Die Vorrichtung 1' kann jedoch auch verwendet werden, um ein ruhendes Objekt 2, 3 zu detektieren.

Indem in der Vorrichtung 1' eine Linie auf der Wand 6 abgescannt wird, kann die Größe des erfassten Objektes anhand der pro abgerasterten Punkt ermittelten Intensitätsänderungen ermittelt werden.

Die für die Anwendung notwendige Abtastfrequenz ergibt sich aus der Entfernung des Lasers zum Messobjekt 2, 3 und dessen Größe. Hierbei ist es erforderlich, dass die Abtastfrequenz so gewählt wird, dass der während eines Zyklus, welcher eine Impulsdauer und eine Pause umfasst, zurückgelegte Weg, kleiner als die Hälfte der Objektgröße ist. Zum Vermessen von Objekten muss die Abtastfrequenz so gewählt werden, dass der während eines Zyklus zurückgelegte Weg kleiner als die Hälfte der geforderten Messgenauigkeit ist.

## Patentansprüche

1. Vorrichtung (1,1') zum Erkennen von Objekten (2, 3) mit einer Lichtquelle und einem Lichtempfänger (5), bei welcher das Objekt (2, 3) in einer in einem lichtdurchlässigen Gefäß (18) vorgesehenen Flüssigkeit (19) nach unten fallbar angeordnet ist, **dadurch gekennzeichnet, dass** die Lichtquelle einer Laserlichtquelle (4) ist, die Vorrichtung (1, 1') eine wenigstens teilweise reflektierende Wand (6) aufweist und zwischen der Laserlichtquelle (4) und der Wand (6) ein Zwischenraum vorgesehen ist, durch den ein Objekt (2, 3) bewegbar oder in dem ein Objekt (2, 3) platzierbar ist, und im Strahlengang zwischen der Laserlichtquelle (4) und der Wand (6) eine bewegbare Spiegeleinrichtung (8) derart angeordnet ist, dass von der Laserlichtquelle (4) abgestrahltes Licht (9) auf die Wand (6) richtbar ist, wobei die Bewegung der Spiegeleinrichtung (8) so steuerbar ist, dass die Wand (6) wenigstens entlang einer Linie mit dem von der Laserlichtquelle (4) abgestrahlten Licht (9) bestrahlbar ist und von der Wand (6) reflektiertes Licht (9) von dem Lichtempfänger (5) detektierbar ist, und wobei die Wand (6) gewölbt ist, wobei die Wand (6) alternierende helle und dunkle Bereiche (10, 11), die ein regelmäßiges Streifenmuster (12) ausbilden, aufweist oder das Licht (9) gepulst ist.

2. Vorrichtung nach Anspruch 1, **dadurch gekennzeichnet, dass** das Gefäß (18) ein Becherglas ist, wobei die gewölbte Wand (6) auf einer Seite des Gefäßes (18) unterhalb einer Ringhalterung (22) vorgesehen ist.

3. Vorrichtung nach Anspruch 3, **dadurch gekennzeichnet, dass** das Objekt (2, 3) eine Kugel ist, die auf einer erweichbaren Schicht in der Ringhalterung (22) gehalten wird, unter welcher im Abstand von 25,0 ± 0,4 mm ein Messbereich zum Erfassen der Kugel vorgesehen ist

4. Vorrichtung nach Anspruch 1, **dadurch gekennzeichnet, dass** die alternierenden hellen und dunklen Bereiche (10, 11) ein senkrechtes Streifenmuster ausbilden.

5. Vorrichtung nach einem der vorhergehenden Ansprüche, **dadurch gekennzeichnet, dass** die Spiegeleinrichtung (8) einen elektronisch steuerbaren Schwingspiegel aufweist.

6. Vorrichtung nach einem der vorhergehenden Ansprüche, **dadurch gekennzeichnet, dass** die Laserlichtquelle (4) und der Lichtempfänger (5) in einem Scanmodul (15) mit einem uncodierten High-Low-Ausgangssignal (16) zusammengefasst sind.

7. Verfahren zum Erkennen von Objekten (2, 3) mit einer Lichtquelle und einem Lichtempfänger (5), bei welchem ein in einer in einem lichtdurchlässigen Gefäß (18) vorgesehenen Flüssigkeit (19) herabfallendes Objekt (2, 3) erfasst wird, **dadurch gekennzeichnet, dass** die Lichtquelle eine Laserlichtquelle (4) ist, von welcher Licht (9) auf eine bewegbare Spiegeleinrichtung (8) gerichtet wird, wobei die Spiegeleinrichtung (8) das Licht (9) auf eine gewölbte, zumindest teilweise reflektierende Wand (6) richtet und die Spiegeleinrichtung (8) dabei so gesteuert wird, dass die Wand (6) wenigstens entlang einer Linie durch das Licht (9) bestrahlt wird, und wobei der Lichtempfänger (5) das von der Wand (6) reflektierte Licht (9) detektiert und dabei ein sich zwischen der Laserlichtquelle (4) und der Wand (6) bewegendes oder platziertes Objekt (2, 3) durch eine Änderung des detektierten, reflektierten Lichts (9) erfasst, und wobei ein auf der Wand (6) vorgesehenes, regelmäßiges Streifenmuster (12) von dem Lichtempfänger (5) mit einer Auswerteeinheit (13) als kontinuierliches, uncodiertes 0-1-Bitmuster (23) erfasst wird oder das Licht (9) gepulst auf die Spiegeleinrichtung (8) gestrahlt wird.

8. Verfahren nach Anspruch 7, **dadurch gekennzeichnet, dass** als Gefäß (18) ein Becherglas verwendet wird, wobei die gewölbte Wand (6) auf einer Seite des Gefäßes (18) unterhalb einer Ringhalterung (22) vorgesehen ist.

9. Verfahren nach Anspruch 8, **dadurch gekennzeichnet, dass** das Objekt (2, 3) eine Kugel ist, die vor dem Herabfallen auf einer Schicht (24) in der Ringhalterung (22) aufliegt, wobei die Schicht (24) bei einer bestimmten Temperatur erweicht wird und die von dem Schichtmaterial (24) umhüllte Kugel nach unten fällt, wobei die Kugel nach einer bestimmten Fallstrecke erfasst wird.

## Claims

1. Apparatus (1, 1') for recognizing objects (2, 3) with a light source and optical receiver (5) in which the object (2, 3) is arranged in a downward falling manner in a liquid (19) provided in a translucent vessel (18) **characterized in that** the light source is a laser light source (4); the apparatus (1, 1') is equipped with an at least partially reflecting wall (6) and a space is provided between the laser light source (4) and the wall (6) through which an object (2, 3) is movable or in which an object (2, 3) is placeable, and a movable mirror device (8) is arranged in the light path between the laser light source (4) and the wall (6) in such a way that the light (9) emitted from the laser light source (4) is directable to the wall (6) where the movement of the mirror device (8) is controllable in such a way that that the wall (6) is radiatable with light (9) emitted from the laser light source (4) at least along one line, and the light (9) reflected by the wall (6) is detectable through the optical receiver (5) and where the wall (6) is domed, and where the wall (6) features alternating bright and dark areas (10, 11) forming a regular strip pattern (12) or the light (9) is pulsed.

2. Apparatus according to Claim 1 **characterized in that** the vessel (18) is a beaker where the domed wall (6) is provided on one side of the vessel (18) below a ring-type fixture (22).

3. Apparatus according to Claim 3 **characterized in that** the object (2, 3) is a ball held in the ring-type fixture (22) on a softenable layer under which a measuring range is provided at a distance of 25.0 ± 0.4 mm to sense the ball.

4. Apparatus according to Claim 1 **characterized in that** the alternating bright and dark areas (10, 11) form a vertical strip pattern.

5. Apparatus according to one of the previous claims **characterized in that** the mirror device (8) has an electronically controllable swivelling reflector.

6. Apparatus according to one of the previous claims **characterized in that** the laser light source (4) and the optical receiver (5) are pooled in a scan module (15) with an uncoded high-low output signal (16).

7. Method for recognizing objects (2, 3) using a light source and an optical receiver (5) in which an object (2, 3) falling downward in a liquid (19) provided in a translucent vessel (18) is sensed, **characterized in that** the light source is a laser light source (4) from which light (9) is directed to a movable mirror device (8) where the mirror device (8) directs the light (9) to a domed, at least partially reflecting wall (6), and the mirror device (8) is controlled in such a way that the wall (6) is exposed to the light (9), at least along one line, and where the optical receiver (5) detects the light (9) reflected by the wall (6) and senses an object (2, 3) moving or placed between the laser light source (4) and the wall (6) through a change of the detected, reflected light (9), and where a regular strip pattern (12) provided on the wall (6) is sensed by the optical receiver (5) using an evaluation unit (13) as a continuous, uncoded 0-1 bit pattern (23) or the light (9) is radiated in a pulsed manner to the mirror device (8).

8. Method according to Claim 7 **characterized in that** a beaker is used as a vessel (18) where the domed wall (6) is provided on one side of the vessel (18) below the ring-type fixture (22).

9. Method according to Claim 8 **characterized in that** the object (2, 3) is a ball resting on a layer (24) in the ring-type fixture (22) prior to falling where the layer (24) is softened at a certain temperature and the ball, covered by the layer material (24), falls down whereby the ball is sensed after a certain falling distance.

## Revendications

1. Dispositif (1, 1') de reconnaissance d'objets (2, 3) avec une source lumineuse et un récepteur de lumière (5), où l'objet (2, 3) est disposé, de telle sorte qu'il puisse tomber vers le bas, dans un liquide (19) qui, lui, se trouve dans un récipient (18) transparent, **caractérisé en ce que** la source lumineuse est une source lumineuse laser (4), que le dispositif (1, 1') est muni d'une paroi (6) qui, au moins partiellement, est réfléchissante, **en ce qu'**un espace par lequel peut être passé un objet (2, 3) ou dans lequel peut être placé un objet (2, 3) est prévu entre la source lumineuse laser (4) et la paroi (6), **en ce qu'**un dispositif réflecteur (8) mobile est placé dans le parcours optique entre la source lumineuse laser (4) et la paroi (6), de telle sorte que la lumière (9) rayonnée par la source lumineuse laser (4) puisse être dirigée vers la paroi (6), le mouvement du dispositif réflecteur (8) étant contrôlé de telle sorte que la paroi (6) puisse être, au moins le long d'une ligne, exposée à la lumière (9) rayonnée par la source lumineuse laser (4) et que la lumière réfléchie (9) par la paroi (6) puisse être détectée par le récepteur de lumière (5), et **en ce que** la paroi (6) est voûtée et que cette paroi (6) présente des zones claires et foncées alternantes (10, 11) qui, elles, forment un dessin à rayures régulières (12), ou que la lumière (9) est pulsée.

2. Dispositif selon la revendication 1, **caractérisé en ce que** le récipient (18) est une coupe, la paroi (6) voûtée étant disposée sur l'un des côtés du récipient (18) et au-dessous d'un support annulaire (22).

3. Dispositif selon la revendication 3, **caractérisé en ce que** l'objet (2, 3) est une boule qui est disposée dans le support annulaire (22), cette boule étant située sur une couche en matière ramollissante, et **en ce qu'**en intervalles de 25,0 ± 0,4 mm, une étendue de mesure permettant de reconnaître la boule est prévue au-dessous du support annulaire.

4. Dispositif selon la revendication 1, **caractérisé en ce que** les zones claires et foncées alternantes (10, 11) forment un dessin à rayures verticales.

5. Dispositif selon l'une quelconque des revendications précédentes, **caractérisé en ce que** le dispositif réflecteur (8) est doté d'un miroir oscillant à contrôle électronique.

6. Dispositif selon l'une quelconque des revendications précédentes, **caractérisé en ce que** la source lumineuse laser (4) et le récepteur de lumière (5) sont réunis dans un module de balayage (15) qui, lui, fonctionne avec un signal de sortie "tout ou rien" non codé (16).

7. Procédé de reconnaissance d'objets (2, 3) avec une source lumineuse et un récepteur de lumière (5), où est reconnu un objet (2, 3) qui est disposé, de telle sorte qu'il puisse tomber vers le bas, dans un liquide (19) qui, lui, se trouve dans un récipient transparent (18), **caractérisé en ce que** la source lumineuse est une source lumineuse laser (4) d'où la lumière (9) est dirigée vers un dispositif réflecteur (8) mobile, ce dispositif réflecteur (8) rayonnant la lumière (9) vers une paroi (6) voûtée qui, elle, est au moins partiellement réfléchissante, **en ce que** le dispositif réflecteur (8) est contrôlé de telle sorte que la paroi (6) soit exposée, au moins le long d'une ligne, aux rayons de lumière (9), **en ce que** le récepteur de lumière (5), détecte la lumière réfléchie par la paroi (6), tout en reconnaissant en même temps un objet (2, 3) qui, lui, est déplacé ou placé entre la source lumineuse laser (4) et la paroi (6), ceci grâce à un changement de direction de la lumière (9) détectée puis réfléchie, et **en ce qu'**un dessin à rayures régulières (12) prévu sur la paroi (6) est reconnu, en tant que séquence binaire 0-1 continue et non codée (23), par le récepteur de lumière (5) qui, lui, est doté d'une unité de saisie (13), ou que la lumière (9) est rayonnée sous forme pulsée vers le dispositif réflecteur (8).

8. Procédé selon la revendication 7, **caractérisé en ce que** le récipient (18) est une coupe et que la paroi (6) voûtée est disposée sur l'un des côtés du récipient (18) et au-dessous d'un support annulaire (22).

9. Procédé selon la revendication 8, **caractérisé en ce que** l'objet (2, 3) est une boule qui, avant de tomber vers le bas, est placée sur une couche (24) et dans un support annulaire (22), et **en ce que** cette couche (24) s'amollit dès qu'une certaine température est atteinte, ce qui fait tomber la boule enveloppée de la matière provenant de la couche (24) vers le bas, la reconnaissance de la boule étant effectuée à un moment déterminé lors de sa chute.
